Europäisches Patentamt

European Patent Office

Office européen des brevets

(11) Publication number: 0 304 867
A2

# EUROPEAN PATENT APPLICATION

(21) Application number: 88113699.8

(22) Date of filing: 23.08.88

(51) Int. Cl.⁴: C07C 45/33 , C07C 47/22 , C07C 51/23 , C07C 57/04 , B01J 23/88 , B01J 23/89 , B01J 27/192

(30) Priority: 26.08.87 JP 210244/87

(43) Date of publication of application: 01.03.89 Bulletin 89/09

(84) Designated Contracting States: BE DE ES FR GB IT NL

(71) Applicant: Nippon Shokubai Kagaku Kogyo Co., Ltd
1, 5-chome, Koraibashi Higashi-ku
Osaka-shi Osaka-fu 541(JP)

(72) Inventor: Kinumi, Kazunori
No. 205, Mezon-Muko 1-25-10,
Mukomoto-machi
Amagasaki-shi Hyogo-ken(JP)
Inventor: Aoki, Yukio
336-8, Tobo Taishi-cho
Ibo-gun Hyogo-ken(JP)
Inventor: Wada, Masahiro
3-23, Matsugaoka-cho
Nishinomiya-shi Hyogo-ken(JP)

(74) Representative: Kraus, Walter, Dr. et al
Patentanwälte Kraus, Weisert & Partner
Thomas-Wimmer-Ring 15
D-8000 München 22(DE)

(54) Process for preparation of methacrolein and methacrylic acid from methyl tert.-butyl ether.

(57) A process for preparation of methacrolein and methacrylic acid characterized by carrying out catalytic gas phase oxidation of methyl tert-butyl ether with a molecular oxygen-containing gas by using a ring-shaped catalyst which has an outer diameter of 3.0 to 10.0 mm, a length of 0.5 to 2.0 times the outer diameter and a through hole, along the length, having a diameter of 0.1 to 0.7 times the outer diameter, and which comprises an active substance having a composition represented by general formula

$$Mo_{(a)}W_{(b)}Bi_{(c)}Fe_{(d)}A_{(e)}B_{(f)}C_{(g)}D_{(h)}O_{(x)}$$

wherein Mo represents molybdenum, W represents tungsten, Bi represents bismuth, Fe represents iron, A represents at least one element selected from the group consisting of nickel and cobalt, B represents at least one element selected from the group consisting of alkali metals, alkaline earth metals and thallium, C represents at least one element selected from the group consisting of phosphorus, tellurium, antimony, tin, cerium, lead, niobium, manganese, zinc, germanium, gallium, lanthanum, indium, cadmium, vanadium, chromium, uranium and silver, D represents at least one element selected from the group consisting of silicon, aluminum, titanium and zirconium and O represents oxygen; and further, each of a, b, c, d, e, f, g, h, and x represents an atomic ratio, when a = 12, then b = 0 to 10, c = 0.1 to 10, d = 0.1 to 20, e = 2 to 20, f = 0 to 10, g = 0 to 4 and h = 0 to 30, and x is a numerical value to be dependent on the valences of the other elements.

This invention relates to a process for preparation of methacrolein and methacrylic acid. More specifically, it relates to a process for industrially advantageous preparation of methacrolein and methacrylic acid by catalytic gas phase oxidation of methyl tert-butyl ether (to be referred to as "MTBE" hereinbelow) with a molecular oxygen-containing gas in the presence of an oxidizing catalyst containing molybdenum, iron, bismuth and cobalt and/or nickel and having a specific ring-like shape.

In the case of use of tertiary butanol as a starting material for preparation of methacrolein by a catalytic gas phase oxidation reaction, many catalysts therefor have also been proposed hitherto.

In general, isobutylene or tertiary butanol has been obtained by subjecting a so-called $C_4$ spent fraction resulting from removal of butadiene from a $C_4$ fraction produced as a by-product at a naphtha cracking step, to treatment for separation thereof by sulfuric acid method, hydration method or a method of going through MTBE obtained by a reaction with methanol. A large amount of MTBE are now in use as a gasoline modifier. Therefore, it is economically advantageous if it is possible to develop a process thereby to directly convert MTBE to mathacrolein or methacrylic acid by a catalytic gas phase oxidation reaction without converting it to isobutylene once as mentioned above.

However, there are only a few literatures on a process for preparation of methacrolein or methacrylic acid from MTBE as a starting material, and one literature which can be referred to is, for example, Japanese Laid-Open Patent Publication No. 18508/1978. The publication discloses a process for preparation of methacrolein which comprises oxidizing a tert-butyl group-containing compound selected from the class consisting of tert-butyl alcohol, alkyl tert-butyl ether in which the alkyl group contains 1 to 4 carbon atoms and a mixture of isobutylene dimer and (or) iso butylene trimer with isobutylene, in the presence of a catalyst containing main components of iron, bismuth and molybdenum, at least one essential subcomponent selected from nickel, cobalt, magnesium, zinc, manganese, cadmium and calcium and an optional component of alkali metals, barium, strontium, thallium, indium, silver, copper or a mixture of these, or phosphorus, antimony, germanium, chromium, thorium, tin, niobium, praseodymium, tungsten, boron, zirconium, cerium, arsenic or a mixture of these.

According to the above Publication, the yield of methacrolein in a single flow is 73 %, and the yield of isobutylene in a single flow is 4 %. However, this catalyst giving these yields is not satisfactory as an industrially usable one, in view of the presently practically utilized process using isobutylene or tertiary butanol as a starting material.

In the case of using MTBE as a starting material, it is very desirable to directly convert it to methyl methacrylate without preconverting it to isobutylene. However, when a catalyst of molybdenum-iron-bismuth type is used, a major part of the resulting reaction product tends to be methacrolein and only a small amount of methacrylic acid tends to be produced. Further, at the same time, methanol is formed as a by-product, and the formed methanol is not desirable for a step of separation and purification of methacrolein and methacrylic acid. The methanol also reacts with acetic acid, maleic acid and other various acids which are simultaneously formed as by-products, to form ester compounds. These disadvantages constitute a large factor preventing industrilization of this process.

It is an object of this invention to provide a process for producing methacrolein and methacrylic acid from MTBE without causing the above disadvantages.

The present inventors have thought it advantageous to effect heat recovery of methanol formed as a by-product in decomposition of MTBE by combusting same on a catalyst, in order to achieve the above object, and further thought it desirable to relieve a thermal load caused by exothermic heat on the catalyst in order to combust the methanol on the catalyst as much as possible. And on the basis of these thoughts, they made studies diligently, and reached this invention.

Thus, according to this invention, there is provided a process for preparation of methacrolein and methacrylic acid characterized by carrying out catalytic gas phase oxidation of methyl tert-butyl ether with a molecular oxygen-containing gas by using a ring-shaped catalyst which has an outer diameter of 3.0 to 10.0 mm, a length of 0.5 to 2.0 times the outer diameter and a through hole, along the length, having a diameter of 0.1 to 0.7 times the outer diameter, and which comprises an active substance having a composition represented by general formula

$$Mo_{(a)}W_{(b)}Bi_{(c)}Fe_{(d)}A_{(e)}B_{(f)}C_{(g)}D_{(h)}O_{(x)}$$

wherein Mo represents molybdenum, W represents tungsten, Bi represents bismuth, Fe represents iron, A represents at least one element selected from the group consisting of nickel and cobalt, B represents at least one element selected from the group consisting of alkali metals, alkaline earth metals and thallium, C represents at least one element selected from the group consisting of phosphorus, tellurium, antimony, tin, cerium, lead, niobium, manganese, zinc, germanium, gallium, lanthanum, indium, cadmium, vanadium, chromium, uranium and silver, D represents at least one element selected from the group consisting of silicon, aluminum, titanium and zirconium and O represents oxygen; and further, each of a, b, c, d, e, f, g, h, and x represents an atomic ratio, when a = 12, then b = 0 to 10, c = 0.1 to 10, d = 0.1 to 20, e = 2 to 20, f = 0 to 10, g = 0 to 4 and h = 0 to 30, and x is a numerical value to be dependent on the valences of the other elements.

It is made possible by the process of this invention to prepare methacrolein and methacrylic acid directly from MTBE at high yields and over a long period of time, further, while the formation of by-products such as methanol, etc., is prevented as much as possible. The above success by the process of this invention is not only because of the composition of the above oxide catalyst but also because largely of the formation of the catalyst in a ring-like shape. The further detailed effects thereof are as follows.

(i) By formation of a catalyst in a ring-like shape, the geometrical surface area of the catalyst increases and according thereto, the conversion of MTBE increases. Methacrolein formed within pores diffuses promptly within the pores as compared with the use of a spherical or cylindrical catalyst, and a reaction into acetic acid, carbon dioxide or carbon monooxide, which is a successive reaction, decreases.

(ii) A pressure drop within catalyst layers decreases, which makes it possible to decrease the energy cost of a blower.

(iii) The temperature in a local abnormal high temperature zone caused by the exothermic reaction can be decreased. More concretely, by formation of a catalyst in a ring-like shape, the temperature-removing effect increases and the temperature in a hot spot decreases. As a result, the increase rate of a pressure drop and the degradation of performance caused by emancipation of molybdenum, one of the catalyst components, during the reaction become small and the life of the catalyst becomes long.

The catalyst of this invention can be prepared by known method. For example, the desired catalyst can be prepared by forming catalyst materials into a powder or clay-like substance by precipitation method, kneading method, etc., optionally adding thereto a small amount of carbon black, stearic acid, starch, polyacrylic acid, mineral oil, stainless powder, whisker, glass fiber, ammonium nitrate, ammonium carbonate, water, etc., forming the mixture into a ring-like shape by using a tablet-forming machine, extruding machine or the like, drying the resultant ring-like product and then firing said product at a temperature of 400 to 700°C under air current or nitrogen current; or by adhering, e.g., by spray method, a slurry of the catalyst components to a carrier having a dimension and shape formed such that the dimension and shape specified by this invention can be finally obtained, drying the resultant ring-like product, and then firing said product at a temperature of 400 to 700°C under air current or nitrogen current.

Recommendable as materials of such a catalyst of this invention are compounds which are decomposable into oxide forms in the above catalyst preparation step. For example, nitrate, ammonium salt, organic acid salt, hydroxide, oxide, metal acid, matal acid ammonium salt, etc., are used.

The alkali metal is selected from lithium, sodium, potassium, rubidium and cesium. The alkaline earth metal is selected from beryllium, magnesium, calcium, barium and strontium.

The catalytic gas phase oxidation reaction according to this invention is carried out by introducing a mixture of 2 to 10 % by volume of MTBE, 3 to 20 % by volume of molecular oxygen, 0 to 60 % by volume of steam and 20 to 80 % by volume of an inert gas (e.g., nitrogen, carbon dioxide gas, methane, ethane, propane, butane, etc.) onto a catalyst prepared in a way along the above-mentioned methods, at a temperature of 250 to 450°C, under pressure of atmospheric pressure to 10 atm and at a space velocity of 300 to 5,000 hr$^{-1}$(STP). The molecular oxygen may be pure oxygen or air. Further, MTBE may be one which is not pure and, for example, may be one having about the same purity as those used as gasoline modifiers.

This invention will be explained hereinbelow according to Examples. However, it shall not be limited thereto. The conversion, selectivity and yield in a single flow are as defined below.

Conversion (%) =

$$\frac{\text{Number of moles of reacted MTBE}}{\text{Number of moles of charged MTBE}} \times 100$$

Selectivity to effective component (%) =

$$\frac{\text{Number of moles of formed effective component}}{\text{Number of moles of reacted MTBE}} \times 100$$

Yield of effective component in a single flow =

$$\frac{\text{Number of moles of formed effective component}}{\text{Number of moles of charged MTBE}} \times 100$$

EXAMPLE 1
---

Cobalt nitrate (1,019 g) and 202 g of ferric nitrate were dissolved in 1,000 ml of water. Separately, 243 g of bismuth nitrate was dissolved in a nitric acid/water solution consisting of 30 ml of concentrated nitric acid and 120 ml of water. Further, separately, 1,059 g of ammonium p-molybdate and 265 g of ammonium p-tungstate were dissolved in 3,000 ml of water, successively, while the water was heated with stirring, and the mixture of the first two aqueous solutions of nitrates was added and mixed with this solution. Then, an aqueous solution of 39 g of cesium nitrate in 100 ml of water and 203 g of silica sol containing 20 % by weight of silica were successively added and mixed therewith. The resultant suspension was heated with stirring, and evaporated and dried to solidness. Then the solid was formed into a ring-like shape having an outer diameter of 5.0 mm, a length of 5.5 mm and a through hole inner diameter of 2.0 mm and the ring-shaped product was fired under air current at 500°C for 6 hours.

The atomic ratio in the composition of the resultant catalyst oxide except oxygen was $Mo_{12}W_{0.5}Co_7Bi_1Fe_1Si_{1.35}Cs_{0.4}$.

The resultant catalyst (1,500 ml) was filled in a steel reaction tube having a diameter of 25.4 mm, and a mixture gas composed of 5 % by volume of MTBE, 13 % by volume of oxygen, 5 % by volume of steam and 77 % by volume of nitrogen was introduced to carry out a reaction under conditions where the reaction temperature was 360°C and the space velocity was 1,000 $hr^{-1}$(STP). Table 1 shows a pressure drop and $\Delta T$ (temperature difference between reaction temperature and hot spot) and yields during the reaction.

In addition, products formed other than those shown in Table 1 were 0.5 mole% of methyl methacrylate, 1.5 mole% of isobutylene and a trace (not more than 0.1 mole %) of methanol. It was also found that a trace of carbon dioxide, carbon monooxide, acetic acid, acetone, acetaldehyde, etc., were formed as by-products.

Further, Table 1 also shows the test results obtained after the reaction was continued for 4,000 hours.

During the above 4,000 hour reaction, it was necessary to elevate the reaction temperature by 5°C. However, at the time when 4,000 hours passed and the reaction was stopped, the reaction system was subject to heat treament at 350°C for 3 hours under air current containing a small amount of steam, and the reaction was restarted to show that the reaction performance was restored to values obtained at the beginning of the reaction.

EXAMPLE 2
---

A part of the suspension obtained in Example 1 was aged at 80°C for 3 hours.

The above suspension was adhered to an alpha-alumina carrier preformed in a ring-like shape having an outer diameter of 8 mm, a length of 8.8 mm and a through hole inner diameter of 4 mm, by spray method under heated air current, and then the sprayed carrier was dried and fired under air current at 500°C for 6 hours.

The produced catalyst had an outer diameter of 9.5 mm, a length of 10.5 mm and a through hole inner diameter of 2 mm.

The resultant catalyst was used for a reaction according to Example 1. However, the reaction temperature was set at 370°C. Table 1 shows the results of the reaction.

## EXAMPLE 3

A catalyst was prepared in the same way along the procedure of Example 1 except that the catalyst was dimensioned and shaped to an outer diameter of 3 mm, a length of 3.3 mm and a through hole inner diameter of 1 mm to carry out the same reaction as in Example 1.

Table 1 shows the results of the reaction.

## EXAMPLE 4

A catalyst was prepared in the same way along the procedure of Example 1 except that the catalyst was dimensioned and shaped to an outer diameter of 8 mm, a length of 8.8 mm and a through hole inner diameter of 4 mm, to carry out the same reaction as in Example 1.

Table 1 shows the results of the reaction.

## COMPARATIVE EXAMPLE

A cylindrically shaped catalyst having an outer diameter of 5.0 mm and a length of 5.5 mm was prepared by using a part of the suspension obtained in Example 1, and a catalytic gas phase oxidation reaction of MTBE was carried out in the same way as in Example 1.

Table 1 shows a pressure drop, $\Delta T$, and yield during the reaction. In this case, since $\Delta T$ was high, the selectivity to methacrolein was low and the presence of methanol in an amount corresponding to 15 % of the thoretically formable amount by decomposition of MTBE was found in a reaction product gas.

Table 1 also shows the catalyst performance after the reaction was carried out for 4,000 hours. During the 4,000 hour reaction in this Example, it was necessary to elevate the reaction temperature by 15°C. Further, it was found that methanol was present in the reaction product gas in an amount corresponding to 18 % of the theoretically formable amount, which thus showed that the catalyst performance on combustion of methanol was further degraded.

## EXAMPLE 5

Cobalt nitrate (873.4 g), 145.4 g of nickel nitrate and 202 g of ferric nitrate were dissolved in 1,000 ml of water. Separately, 243 g of bismuth nitrate was dissolved in a nitric acid/water solution consisting of 30 ml of concentrated nitric acid and 120 ml of water. Further, separately, 1,059 g of ammonium p-molybdate and 133 g of ammonium p-tungstate were dissolved in 3,000 ml of water, successively, while the water was heated with stirring, and the mixture of the first two aqueous solutions of nitrates was added and mixed with this solution. And then an aqueous solution of 19.5 g of cesium nitrate, 2.8 g of potassium nitrate and 4.2 g of sodium nitrate in 100 ml of water and 203 g of silica sol containing 20 % by weight of silica were added and mixed therewith successively. The resultant suspension was heated with stirring, and evaporated and dried to solidness. Then the solid was formed into a ring-like shape having an outer diameter of 5 mm, a length of 5.5 mm and a through hole inner diameter of 2.0 mm and the ring-shaped product was fired under air current at 500°C for 6 hours.

The atomic ratio in the composition of the resultant catalyst oxide except oxygen was
$Mo_{12}W_1Co_6Ni_1Fe_1Si_{1.35}Cs_{0.2}Na_{0.1}K_{0.1}$.

The resultant catalyst (1,500 ml) was filled in a steel reaction tube having a diameter of 25.4 mm, and a mixture gas composed of 5 % by volume of MTBE, 13 % by volume of oxygen, 5 % by volume of steam, 20 % by volume of propane, 20 % by volume of carbon dioxide gas and 37 % by volume of nitrogen was introduced to carry out a reaction under conditions where the reaction temperature was 360 °C and the space velocity was 1,000 $hr^{-1}$(STP). Table 1 shows a pressure drop, $\Delta T$, and yields during the reaction.

## EXAMPLE 6

Example 1 was repeated except that 11.6 g of rubidium carbonate and 26.6 g of thallium nitrate were used in place of cesium nitrate, to prepare a catalyst.

The atomic ratio in the composition of the resultant catalyst oxide except oxygen was $Mo_{12}W_2Co_7Bi_1Fe_1Si_{1.35}Rb_{0.2}Tl_{0.2}$.

Table 1 shows the results of the same reaction as in Example 1 by using this catalyst.

## EXAMPLE 7

Cobalt nitrate (1,019 g), 145.4 g of nickel nitrate and 808 g of ferric nitrate were dissolved in 1,000 ml of water. Separately, 364.5 g of bismuth nitrate was dissolved in a nitric acid/water solution consisting of 45 ml of concentrated nitric acid and 180 ml of water. Further, separately, while 3,000 ml of water was heated with stirring, 1,059 g of ammonium p-molybdate was dissolved in the water, and further 43.4 g of cerous nitrate was added. And the mixture of the first two aqueous solutions of nitrates was added dropwise and mixed with this solution. And then an aqueous solution of 25.6 g of magnesium nitrate in 100 ml of water and 39.9 g of titanium dioxide were added and mixed therewith successively. The resultant suspension was heated with stirring, and evaporated and dried to solidness. Then the solid was formed into a ring-like shape having an outer diameter of 5 mm, a length of 5.5 mm and a through hole inner diameter of 2.0 mm and the ring-shaped product was fired under air current at 500 °C for 6 hours.

The atomic ratio in the composition of the resultant catalyst oxide except oxygen was $Mo_{12}Co_7Ni_1Bi_{1.5}Fe_4Ti_{1.0}Ce_{0.2}Mg_{0.2}$.

The oxidation reaction of MTBE was carried out according to the procedure of Example 1. Table 1 shows the results of the reaction.

## EXAMPLE 8

Example 7 was repeated except that 23.6 g of calcium nitrate and 10.6 g of strontium nitrate were used in place of magnesium nitrate, that 7.5 g of tin oxide and 7.3 g of antimony trioxide were used in place of cerous nitrate and that 61.6 g of zirconium oxide was used in place of titanium dioxide. And the reaction test was carried out also according to the procedure of Example 7.

The atomic ratio in the composition of the resultant catalyst oxide except oxygen was $Mo_{12}Co_7Ni_1Bi_{1.5}Fe_4Zr_{1.0}Sn_{0.1}Sb_{0.1}Ca_{0.2}Sr_{0.1}$.

Table 1 shows the results.

## EXAMPLE 9

Cobalt nitrate (1,019 g) and 303 g of ferric nitrate were dissolved in 1,000 ml of water. Separately, 243 g of bismuth nitrate was dissolved in a nitric acid/water solution consisting of 30 ml of concentrated nitric acid and 120 ml of water. Further, separately, while 3,000 ml of water was heated with stirring, 1,059 g of ammonium p-molybdate was dissolved in the water, and further 11.7 g of ammonium m-vanadate and 6.3 g of ammonium bichromate were added. And the mixture of the first two aqueous solutions of nitrates was added dropwise and mixed with this solution. And then 13.1 g of barium nitrate and 1.2 g of beryllium oxide were added, and further 187.6 g of aluminum nitrate was added and mixed. The resultant suspension was heated with stirring, and evaporated and dried to solidness. Then the solid was formed into a ring-like

6

shape having an outer diameter of 5 mm, a length of 5.5 mm and a through hole inner diameter of 2.0 mm and the ring-shaped product was fired under air current at 500°C for 6 hours.

The atomic ratio in the composition of the resultant catalyst oxide except oxygen was $Mo_{12}Co_7Fe_{1.5}Bi_{1.0}V_{0.2}Cr_{0.1}Ba_{0.1}Be_{0.1}Al_{1.0}$.

The oxidation reaction of MTBE was carried out according to the procedure of Example 1. Table 1 shows the results of the reaction.

## EXAMPLE 10

A catalyst was prepated by repeating the procedure of Example 9 except that 4.9 g of orthophosphoric acid and 8.0 g of tellurium dioxide were used in place of ammonium m-vanadate.

The atomic ratio in the composition of the resultant catalyst oxide except oxygen was $Mo_{12}Co_7Fe_{1.5}Bi_{1.0}P_{0.1}Te_{0.1}Cr_{0.1}Ba_{0.1}Be_{0.1}Al_{1.0}$.

The oxidation reaction of MTBE was carried out according to the procedure of Example 1. Table 1 shows the results of the reaction.

## EXAMPLE 11

Cobalt nitrate (1,019 g) and 202 g of ferric nitrate were dissolved in 1,000 ml of water. Separately, 243 g of bismuth nitrate was dissolved in a nitric acid/water solution consisting of 30 ml of concentrated nitric acid and 120 ml of water. Further, separately, while 3,000 ml of water was heated with stirring, 1,059 g of ammonium p-molybdate and 265 g of ammonium p-tungstate were dissolved in the water, successively, and the mixture of the first two aqueous solutions of nitrates was added dropwise and mixed with this solution. And then an aqueous solution of 39 g of cesium nitrate, 16.6 g of lead nitrate and 14.9 g of zinc nitrate in 150 ml of water, 13.3 g of niobium pentoxide and 203 g of silica sol containing 20 % by weight of silica were added, respectively, and mixed. The resultant suspension was heated with stirring, and evaporated and dried to solidness. Then the solid was formed into a ring-like shape having an outer diameter of 5 mm, a length of 5.5 mm and a through hole inner diameter of 2.0 mm and the ring-shaped product was fired under air current at 500°C for 6 hours.

The atomic ratio in the composition of the resultant catalyst oxide except oxygen was $Mo_{12}W_{0.5}Co_7Bi_1Fe_1Si_{1.35}Pb_{0.1}Zn_{0.1}Nb_{0.1}Cs_{0.4}$.

The oxidation reaction of MTBE was carried out according to the procedure of Example 1. Table 1 shows the results of the reaction.

## EXAMPLE 12

A catalyst was prepared by repeating the procedure of Example 11 except that 28.7 g of manganese nitrate, 8.5 g of silver nitrate and 5.2 g of germanium oxide were used in place of lead nitrate, zinc nitrate and niobium pentoxide, respectively.

The atomic ratio in the composition of the resultant catalyst oxide except oxygen was $Mo_{12}W_{0.5}Co_7Bi_1Fe_1Si_{1.35}Mn_{0.2}Ag_{0.1}Ge_{0.1}Cs_{0.4}$.

The oxidation reaction of MTBE was carried out according to the procedure of Example 1. Table 1 shows the results of the reaction.

## EXAMPLE 13

A catalyst was prepared by repeating the procedure of Example 11 except that 15.4 g of cadmium nitrate, 3.0 g of indium nitrate and 25.1 g of uranyl nitrate were used in place of lead nitrate, zinc nitrate and niobium pentoxide, respectively.

EP 0 304 867 A2

The atomic ratio in the composition of the resultant catalyst oxide except oxygen was
$Mo_{12}W_{0.5}Co_7Bi_1Fe_1Si_{1.35}Cd_{0.1}In_{0.1}U_{0.1}Cs_{0.4}$.
The oxidation reaction of MTBE was carried out according to the procedure of Example 1. Table 1 shows the results of the reaction.

## EXAMPLE 14

A catalyst was prepared by repeating the procedure of Example 11 except that lead nitrate was not used, that 3.6 g of lanthanum nitrate was used in place of zinc nitrate and that 4.7 g of garium oxide was used in place of niobium pentoxide.
The atomic ratio in the composition of the resultant catalyst oxide except oxygen was
$Mo_{12}W_{0.5}Co_7Bi_1Fe_1Si_{1.35}Ga_{0.1}La_{0.1}Cs_{0.4}$.
The oxidation reaction of MTBE was carried out according to the procedure of Example 1. Table 1 shows the results of the reaction.

## Table - 1

| | | MTBE Conversion ratio (%) | Selectivity (%) | | Yield in single flow (%) | | $\Delta T$ (°C) | Pressure drop (mmHg) | Remarks |
|---|---|---|---|---|---|---|---|---|---|
| | | | Methacrolein | Methacrylic acid | Methacrolein | Methacrylic acid | | | |
| Example | 1 | 98.0 | 81.6 | 4.5 | 80.0 | 4.4 | 75 | 90 | |
| | | 97.5 | 82.5 | 4.0 | 80.4 | 3.9 | 73 | 92 | After 4,000 hours |
| | 2 | 98.3 | 83.0 | 4.0 | 81.6 | 4.0 | 70 | 90 | |
| | 3 | 99.7 | 78.6 | 6.3 | 78.4 | 6.3 | 82 | 125 | |
| | 4 | 95.4 | 79.2 | 5.1 | 75.5 | 4.8 | 64 | 51 | |
| Comparative Example | | 97.6 | 75.1 | 4.5 | 73.3 | 4.4 | 87 | 115 | |
| | | 92.1 | 74.6 | 4.2 | 68.7 | 3.9 | 84 | 120 | After 4,000 hours |
| Example | 5 | 99.2 | 77.6 | 5.0 | 77.0 | 5.0 | 70 | 87 | |
| | 6 | 97.6 | 78.2 | 5.1 | 76.3 | 5.0 | 77 | 92 | |
| | 7 | 97.0 | 79.3 | 4.2 | 76.9 | 4.1 | 76 | 90 | |
| | 8 | 98.1 | 77.1 | 5.0 | 75.6 | 4.9 | 75 | 90 | |
| | 9 | 99.3 | 78.6 | 4.0 | 78.0 | 4.0 | 76 | 92 | |
| | 10 | 97.9 | 77.9 | 4.6 | 76.3 | 4.5 | 72 | 87 | |
| | 11 | 98.5 | 81.0 | 4.2 | 79.8 | 4.1 | 71 | 91 | |
| | 12 | 98.0 | 80.1 | 4.5 | 78.5 | 4.4 | 72 | 90 | |
| | 13 | 97.2 | 80.2 | 3.6 | 77.9 | 3.5 | 70 | 93 | |
| | 14 | 97.8 | 80.6 | 4.2 | 78.8 | 4.1 | 74 | 90 | |

## Claims

1. A process for preparation of methacrolein and methacrylic acid characterized by carrying out catalytic gas phase oxidation of methyl tert-butyl ether with a molecular oxygen-containing gas by using a ring-shaped catalyst which has an outer diameter of 3.0 to 10.0 mm, a length of 0.5 to 2.0 times the outer diameter and a through hole, along the length, having a diameter of 0.1 to 0.7 times the outer diameter, and which comprises an active substance having a composition represented by general formula

$$Mo_{(a)}W_{(b)}Bi_{(c)}Fe_{(d)}A_{(e)}B_{(f)}C_{(g)}D_{(h)}O_{(x)}$$

wherein Mo represents molybdenum, W represents tungsten, Bi represents bismuth, Fe represents iron, A represents at least one element selected from the group consisting of nickel and cobalt, B represents at least one element selected from the group consisting of alkali metals, alkaline earth metals and thallium, C represents at least one element selected from the group consisting of phosphorus, tellurium, antimony, tin, cerium, lead, niobium, manganese, zinc, germanium, gallium, lanthanum, indium, cadmium, vanadium, chromium, uranium and silver, D represents at least one element selected from the group consisting of silicon, aluminum, titanium and zirconium and O represents oxygen; and further, each of a, b, c, d, e, f, g, h, and x represents an atomic ratio, when $a = 12$, then $b = 0$ to 10, $c = 0.1$ to 10, $d = 0.1$ to 20, $e = 2$ to 20, $f = 0$ to 10, $g = 0$ to 4 and $h = 0$ to 30, and x is a numerical value to be dependent on the valences of the other elements.

2. A process according to claim 1 wherein the catalyst is formed in a ring-like shape.

3. A process according to claim 1 wherein the catalyst is supported on a ring-like carrier.

4. A process according to claim 1 wherein a mixture composed of 2 to 10 % by volume of methyl tert butyl ether, 3 to 20 % by volume of molecular oxygen, 0 to 60 % by volume of steam and 20 to 80 % by volume of an inert gas is introduced onto the catalyst at a temperature of 250 to 450° C, under a pressure of atmospheric pressure to 10 atm and at a space velocity of 300 to 5,000 hr⁻¹(STP).